# EUROPEAN PATENT SPECIFICATION

(11) **EP 2 632 500 B1**
(45) Date of publication and mention of the grant of the patent: **30.08.2017**
(21) Application number: 11836715.0
(22) Date of filing: 31.10.2011
(51) Int. Cl.: A61L 15/16, A61F 13/42, A61B 5/02

(54) **DETECTION OF BLOOD LEAKAGE BY DETECTING A VOLATILE AGENT**
NACHWEIS VON BLUTLECKS DURCH ERKENNUNG EINES FLÜCHTIGEN WIRKSTOFFS
DÉTECTION DE SAIGNEMENT PAR LA DÉTECTION D'UN AGENT VOLATIL

(30) Priority: 29.10.2010 SE 1001065
(43) Date of publication of application: 04.09.2013
(73) Proprietor: Redsense Medical AB, 302 41 Halmstad (SE)
(72) Inventor: ENGVALL, Daniel, S-302 93 Halmstad (SE); STRÖMSTEN, Patrik, S-435 35 Mölnlycke (SE)
(74) Representative: Asketorp, Göran
(86) International application number: PCT/SE2011/000197
(87) International publication number: WO 2012/057673

(56) References cited:
- EP-A1- 0 370 600
- WO-A1-96/39923
- WO-A1-97/29735
- US-A- 5 643 589
- US-A- 5 741 509
- US-A1- 2004 147 038
- US-A1- 2005 165 370
- US-A1- 2006 172 002
- US-A1- 2007 023 627
- US-A1- 2007 041 935
- US-A1- 2007 142 799
- US-A1- 2008 056 946
- US-A1- 2010 152 683
- US-B1- 6 312 918
- Anonymous: "Lutrasil - skin-friendly and extremely soft spunlaid", Lutrasil , 3 April 2014 (2014-04-03), XP002722743, Retrieved from the Internet: URL:http://www.freudenberg-nw.com/en/Compa ny/Brands/Pages/Lutrasil.aspx [retrieved on 2014-04-03]

## Description

### FIELD OF THE INVENTION

The present invention relates to a method and a system for detecting blood leakage from a wound.

### BACKGROUND OF THE INVENTION

A well-recognized problem in hospital care is that wounds caused by surgery or accidents, in spite of having been properly closed and dressed, may start to bleed again. Due to the dressing by which the wound is covered or due to the patient being unconscious or otherwise unable to recognize the bleeding, it is only noticed by the staff and taken care of after a while. In the meantime the patient may have lost a substantial volume of blood. This will no doubt have a detrimental effect on his or her recovery.

Another problem of similar kind is quite frequently seen in blood dialysis. In a life saving treatment patients with impaired or non-existing renal function purify their blood from salts, urea and other metabolic degradation products on a regular basis, such as two or three times per week. In blood dialysis an artery is punctured by a cannula or needle to make a portion of the patient's blood pass through a dialysis apparatus in which it is purified. The purified blood is returned to the patient by venous infusion through a cannula inserted into a large vein. Most often arterio-venous fistula (or a corresponding graft) is created at a patient's wrist or upper arm, from which blood is removed by an arterial cannula and returned downstream by a venous cannula.

A cannula of this kind usually comes with wings extending from a short cylindrical plastic tube in which the cannula is mounted. These wings can be used for securing the cannula by adhesive tape to prevent it from longitudinal displacement in the vein, fistula, or graft. The adhesive tape may accidentally come off and the cannula withdrawn. Inevitably this results in immediate bleeding, which may be quite severe. If the bleeding is not noticed and stopped at once the patient may lose a large volume of blood. Since dialysis patients are usually anaemic, they are particularly affected by such a loss. In addition it is important to prevent blood contained in the dialysis apparatus from being lost if a cannula is removed accidentally. To cope with a loosening arterial needle a safety means is included in known dialysis apparatus. The safety means comprises a pressure sensor disposed on the input side of the apparatus. If the sensor detects a sudden drop in pressure during dialysis the flow of blood through the apparatus is immediately stopped and the personnel alarmed. Due to the pressure drop in the venous needle a loosening thereof cannot be monitored easily in a corresponding manner.

WO 06/001759 A1 discloses a method for detecting blood leakage from a wound in which a sharp bend of an optical fiber is disposed at the wound. Light passing through the fiber is detected at one end of the fiber. Leaking blood contacting the fiber attenuates the passing light. The attenuation, which indicates blood leakage, is detected and an alarm is triggered. The method of WO 06/001759 A1 employs a device comprising an optical fiber and a medical patch. For hygienic reasons the device is disposable.

US 5643589 discloses a pharmaceutical composition for use in treating an abnormal skin condition, such as a wound, a burn or a blister, in an animal or human. The pharmaceutical composition has an antiseptic or antimicrobial agent and a desiccant for adsorbing moisture or liquid, in and around the area of the abnormal skin, in a form such that the adsorbed moisture or liquid is not readily available to the skin.

US 6312918 discloses a simple examination method of infection with Helicobacter pylori possibly presenting in a gastric mucosa, and a device therefor. The examination method is conducted by collecting gas in gastric cavity, and measuring mainly ammonia and additionally organic amines which are generated due to activities of the bacilli. The measurement is carried out by leading the gas in gastric cavity into oral cavity by vomiting-reflex, and sucking the gas by a metering suction pump through a gas detection tube to read-out a length of color-changed area in the gas detection tube.

The provision of alternative methods of detecting blood leakage from a wound not employing a disposable device or a disposable device, which is more economical and/or simpler to manufacture than the prior art device is desirable.

### OBJECTS OF THE INVENTION

It is an object of the invention to provide a method and a system of the aforementioned kind, which is safe.

It is another object of the invention to provide a method and a system of the aforementioned kind, of which a disposable device is more economical to manufacture than devices known in the art.

Further objects of the invention will become evident from the following summary of the invention, the description of preferred embodiments thereof illustrated in a drawing, and the appended claims.

### SUMMARY OF THE INVENTION

According to an aspect of the invention, there is provided a method of detecting bleeding from a wound, comprising: providing, on a support, a substantially non-volatile agent capable of forming a volatile agent on contact with blood; disposing the non-volatile agent on the support at or near the wound; providing a probe for detection of the volatile agent; disposing the probe for detection of the volatile agent at a distance from the support; generating a flow of air in a direction from the support to the probe; monitoring the formation of volatile agent by the probe, thereby detecting a bleeding of the wound. The non-volatile agent may be included in the group comprising: calcium hydride, barium hydride, calcium carbide, borohydride such as sodium borohydride and potassium borohydride, sodium sulfide, ammonium salt, such as ammonium carbonate, ammonium sulfate, ammonium chloride, orthoester such as triethyl carbonate, tripropyl carbonate, NH3BH3, mixed anhydride of ion exchange resin with carboxylic groups and low-molecular carboxylic acid. There may further be included an acid, a base, or a catalyst promoting the reaction of non-volatile agent with blood. The base may be included in the group of: alkali carbonate, such as lithium carbonate, sodium carbonate, potassium carbonate; alkali hydroxide, such as lithium hydroxide, sodium hydroxide, potassium hydroxide; di- and trialkali phosphate, such as disodium hydrogen phosphate and trisodium phosphate; calcium hydroxide. The volatile agent may be included in the group consisting of: hydrogen, acetylene, methanol, ethanol, hydrogen sulfide, ammonia, C1-C3 carboxylic acid.

In another aspect, there is provided a system for detecting bleeding from a wound, comprising: a support configured to be attached to a skin of a patient at or in the immediate vicinity of the wound; a substantially non-volatile agent provided on the support, the non-volatile agent being disposed at or near the wound and being capable of forming a volatile agent on contact with blood. The system further comprises: a gas detection probe for detection of the volatile agent wherein the probe is disposed at a distance from the support; an electrically driven fan for generating a flow of air in a direction from the support to the probe; and a guiding means for guiding said flow of air in a selected direction towards said probe, said guiding means comprising a tube, wherein the electrically driven fan is mounted in the lumen of the tube; whereby said probe is configured to emit or increase or decrease an electric signal when contacted by the volatile agent as an indication of bleeding of the wound. The non-volatile agent may be included in the group consisting of: calcium hydride, barium hydride, calcium carbide, borohydride such as sodium borohydride and potassium borohydride, sodium sulfide, ammonium salt, such as ammonium carbonate, ammonium sulfate, ammonium chloride, orthoester such as triethyl carbonate, tripropyl carbonate, NH3BH3, mixed anhydride of ion exchange resin with carboxylic groups and low-molecular carboxylic acid. In an embodiment, there may be included any of acid, base, catalyst on a layer of the support and said non-volatile agent on another layer of the support. The layer comprising any of an acid, a base, or a catalyst may be separated from the layer comprising the non-volatile agent by an empty layer. In a further embodiment, the support may be made of a textile material, wherein the textile material is included in the group of: cotton, linen, polymer fiber, cellulose fiber. The support may comprise two or more layers of textile material. The two or more layers may be bonded.

### BRIEF DESCRIPTION OF THE DRAWINGS

The invention will now be explained in more detail by reference to preferred embodiments thereof illustrated in a drawing.
Fig. 1 is a schematic top view of the system of the invention mounted at a person's anterior forearm region, partially sectioned;
Figs. 2 - 5 are radially sectioned views of a layered support loaded with the substantially non-volatile agent of the invention.

### DESCRIPTION OF EMBODIMENTS

The invention will be explained by reference to a wound caused by insertion of a cannula into a vein. The teaching of the invention is however equally applicable to other kinds of wounds, both surgically and accidentally caused wounds, that have stopped bleeding but are at risk of reopening and bleeding.

The present invention is based on the insight that detection of a volatile agent formed by leaking blood contacting a substantially non-volatile agent can be used for this purpose.

More specifically, the present invention is based on the insight that a volatile agent can be formed by the reaction of blood with an agent of low volatility, in the following called non-volatile agent, and that the formation of the volatile agent can be detected by a detection means disposed at a distance from the site of formation. The volatile agent is formed by reaction of the water component of blood with the non-volatile agent. In this invention, the non-volatile agent is an agent capable of forming a volatile agent from which it differs in boiling point by at least 100 °C, and even by 200 °C or 300 °C or more. The property of low volatility of the non-volatile agent is important, since evaporation of the agent, at least of a substantial amount of it, should be avoided during the monitoring period to avoid compromising monitoring safety. In respect of hemodialysis a monitoring period is, for instance, 5 hours or 7 hours. The amount of non-volatile agent evaporated during the monitoring period should not exceed 20 %, preferably it should be less than 10 %, most preferred less than 1 %.

The volatile agent is transported by diffusion/convection to the detection means. Preferably it is transported to the detection means by controlled convection. Controlled convection is convection controlled in a manner so as to create an air stream from the site of formation of the volatile agent to the site of detection can be produced by a convection producing means. The air stream carries transports the volatile agent from the site of formation to the site of detection. A suitable convection producing means comprises a fan, in particular an electrically driven fan. The convection producing means of the invention may also comprise an air stream conducting means such as a tube disposed intermediate between the site of formation and the site of detection.

The volatile agent can be any agent capable of being transported by an air stream in a gaseous state except for a component of air comprised by oxygen, nitrogen, carbon dioxide, carbon monoxide, water, helium, and argon. While air does additionally contain a great number of other components in amounts varying in respect of the particular environment, such as nitrogen oxides, hydrogen, and methane, their low concentration can be compensated for by setting an appropriate lower concentration limit for detection.

The non-volatile agent is disposed at a wound, such as a wound caused by the insertion of a cannula into a blood vessel, so that it is contacted by blood leaking from the wound within a short period of time upon the onset of leakage, such a within less than 30 seconds and even within less than 10 or 5 seconds. A volatile agent so produced can be detected by a detector sensitive to the agent but essentially insensitive to a component of air or to the volatile agent below a selected detection limit.

Examples of suitable non-volatile agents of the invention are (respective volatile agent formed by contact with an aqueous media such as blood in parenthesis):
CaC₂ (acetylene);
CaH₂ (hydrogen):
BaH₂ (hydrogen);
NaBH₄ (hydrogen);
KBH₄ (hydrogen);
NaBH₄ or KBH₄ + catalyst, such as a carboxylic acid, CoCl₂, aluminum oxide (hydrogen);
NH₃BH₃ + catalyst, such as Pd²⁺ or cation exchange resin, such as amberlite (hydrogen) (NH₄)₂CO₃ (ammonia);
NH₄Cl + base, for instance Na₂CO₃ (ammonia);
(NH₄)₂ SO₄ + base, for instance Na₂CO₃ (ammonia)
a mixed anhydride of an ion exchange resin with carboxylic groups and a low-molecular carboxylic acid, in particular acetic acid (low molecular carboxylic acid);
triethyl carbonate + acid, for instance citric acid (ethanol);
tripropyl carbonate + acid, for instance citric acid (propanol);
Na₂S + acid, for instance NaH₂PO₄ (hydrogen sulfide).

The non-volatile agent or a matrix comprising the non-volatile agent is attached to a support, in particular a textile woven or non-woven support, such as a cotton pad, a cellulose fiber pad, a synthetic polymer fiber pad, including a pad comprising two or more fiber materials. The pad is attached to the skin at the wound, that is, preferably not in contact with the wound but disposed in its immediate vicinity, such as surrounding the wound fully or partially. Attachment of the pad to the skin can be by, for instance, a medical-grade adhesive.

The catalyst or acid or base can be applied to the support in a manner so as to keep it separate from the non-volatile agent until the support is contacted by blood. An appropriate support is a layered support comprising one or more layers of non-volatile agent and one or more layers of catalyst or base. A layer comprising any of non-volatile agent, catalyst, acid, and base can be protected by disposing it between empty layers.

Alternatively or additionally, the non-volatile agent can be entrapped in a matrix, such as a carbohydrate matrix, or enclosed in a shell readily dissolvable in water, such as a shell of gelatin.

Gas detectors suitable for detection of the volatile agent of the invention are known in the art. Particularly useful are semiconductor based detectors comprising surfaces capable of selective adsorption of the volatile agent.

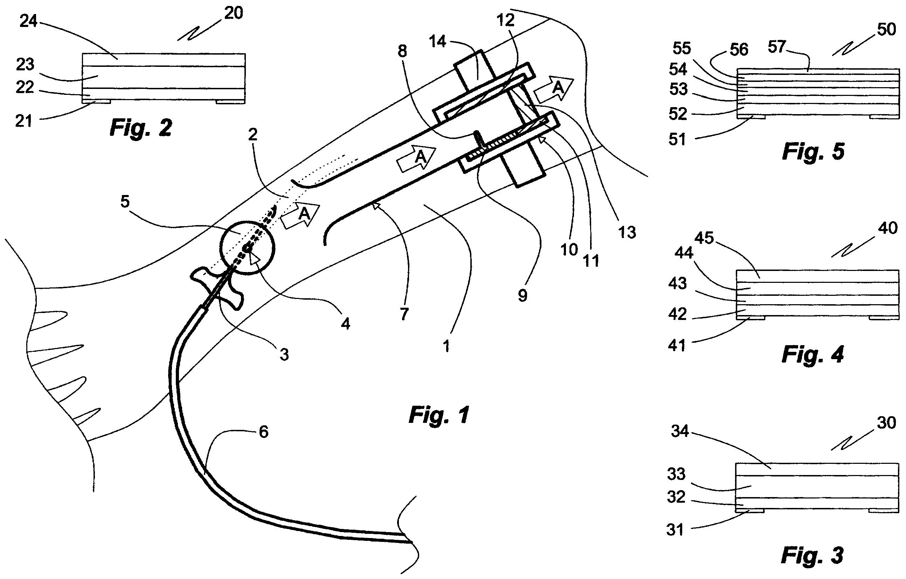

By means of a strap 14 provided with a Velcro® closure (not shown) extending around a patient's forearm the system of the invention shown in Fig. 1 is mounted at the anterior forearm region 1 for detection of a leakage of blood from a wound 4 caused by the insertion of a cannula 3 into a vein 2, such as at an arterial-venous anastomosis. The cannula 3 is connected with a dialyzer (not shown) by means of a flexible tube 6. A layered support 5 loaded with non-volatile agent of the invention is disposed centered above the wound 4. The circular support 5 comprises a top face and a bottom face. The circumference of the bottom face is provided with a medical adhesive by which the support 5 is attached to the skin. The air intake opening of an air stream conducting tube 7 is disposed at a short distance from the support 5 in a cranial direction. A gas detection probe 8 extends into the lumen of the tube 7 near the output opening thereof. The gas detection probe 8 pertains to a gas detector 9 disposed between a casing 10 enclosing a cranial terminal portion of the tube 7 and said terminal portion. The gas detection probe 8 is selectively sensitive to a volatile agent other than a natural component of air (nitrogen, oxygen, carbon dioxide, helium, neon, argon, xenon). When contacted by an agent to which it is sensitive, the probe emits or increases or decreases an electrical signal, which is received and analyzed by the gas detector 9. While trace amounts of hydrogen, hydrogen sulfide, and ammonia can be present in air, their presence is disregarded from or compensated for in the present context. The gas detector 9 comprises a microprocessor and software dedicated to the analysis of the gas detection probe 8 signal. The gas detector 9 including the probe 8 is powered by a battery 12, such as a rechargeable lithium ion battery. Proximally of the probe 8 a fan 13 powered by a DC motor is mounted in the lumen of the tube 7. The fan 13 creates a cranially directed air stream A in the tube 7. Air is sucked in at the air intake opening and expelled at the air output opening after the air stream has passed the gas detection probe 8. A volatile agent released at or from the support 5 will be sucked into the caudal opening of the tube 7 by the air stream generated by the fan 13 and made pass the gas detection probe 8. If the volatile agent is one that has affinity to the gas detection probe 8, that is, is selectively adsorbed by a surface of the probe 8, an electrical signal of the probe 8 is created or amplified or attenuated. The signal or change in signal strength is detected by the detector 9. If the signal or change in signal strength exceeds a preset limit, an alarm produced by an alarm unit 11 is triggered. The alarm may be of a visual and/or audible nature. The visual or audible alarm can be emitted by the alarm unit 11. Alternatively or additionally, the alarm unit 11 can comprise a sender for transmission of a wireless signal to a receiver (not shown) comprising visual and/or audible alarm generating means like a flashing light or a bell or similar. The alarm signal can also be transmitted to a control room from where one or several persons are supervising dialysis patients during dialysis. Alternatively or additionally, the alarm unit 11 can be electrically connected with the dialysis machine (not shown) to which the patient is coupled via one or two cannulae, only one cannula being shown in Fig. 1. This connection allows to stop dialysis as soon as the alarm is triggered.

Figs. 2 to 5 show supports according to the invention in radial section. Their circular bottom face is provided with a peripheral layer of medical adhesive 21, 31, 41, 51 and so defined. The layers are numbered starting from the bottom layer.

The support 20 of Fig. 2 comprises three layers of non-woven cotton bonded by a polymer melt applied at intersections of a square net pattern. Layers 22 and 24 are empty protective layers. The intermediate layer 23 comprises calcium hydride flakes of a minimum sieved size so as to be firmly held by the cotton threads. Blood reaching the intermediate layer via bottom layer 22 reacts with calcium hydride under formation of hydrogen, which can be detected by a hydrogen-specific gas detection probe.

The support 30 of Fig. 3 comprises a bottom layer 32 and a top layer 34 layers of non-woven cotton interspaced by a central layer 32 of cellulose acetate fiber. The central layer comprises calcium carbide made adherent to the fibers by contacting it with a solution of polyvinyl chloride-vinyl acetate copolymer dissolved in tetrahydrofuran.
Blood reaching the intermediate layer 33 via the bottom layer 32 reacts with calcium carbide under formation of acetylene, which can be detected by an acetylene-specific gas detection probe.

The support 40 of Fig. 4 comprises a bottom layer 42 and a top layer 45 of non-woven cotton interspaced by two layers 43,44 of same material. Layer 43 comprises citric acid and heparin made to adhere to the fiber by aqueous carboxymethyl cellulose. Layer 44 comprises sodium borohydride deposed on the fiber as from a solution in diglyme. The layers are not bonded; they are kept in the illustrated layered configuration by encasement in form of a net of polypropylene fibers (not shown). Blood reaching the citric acid layer 43 via the bottom layer 42 dissolves the citric acid, the aqueous solution of which is transported to adjacent layer 44 where sodium borohydride is dissolved and reacted with water under formation of hydrogen, which can be detected by a hydrogen-specific gas detection probe.

The support of Fig. 5 comprises a bottom layer 52 and a top layer 57 of a cotton web. The layer 53 abutting the bottom layer is of fluffy non-woven cotton soaked with an aqueous solution of NaH₂PO₄ and heparin, then thoroughly dried. The layer 54 on top of the sodium dihydrogen phosphate layer 53 is of an empty cotton web. The layer 55 disposed on top of the layer 54 is of fluffy non-woven cotton soaked with an aqueous solution of sodium sulfide and then thoroughly dried. Blood entering from below the bottom layer 52 and from there the sodium dihydrogen phosphate layer dissolves NaH₂PO₄ under formation of an acidic aqueous solution which, after passage through the empty separating layer 54 enters the sodium sulfide layer 56 and dissolves sodium sulfide which is decomposed in the acidic media to hydrogen sulfide and disodium hydrogen phosphate. Hydrogen sulfide evolves as a gas from the solution and can be detected by a hydrogen sulfide-specific gas detection probe.

## Claims

1. A method of detecting bleeding from a wound, comprising: providing, on a support, a substantially non-volatile agent capable of forming a volatile agent on contact with blood; disposing the non-volatile agent on the support at or near the wound; providing a probe for detection of the volatile agent; disposing the probe for detection of the volatile agent at a distance from the support; generating a flow of air in a direction from the support to the probe; monitoring the formation of volatile agent by the probe, thereby detecting a bleeding of the wound.

2. The method of claim 1, wherein the non-volatile agent is included in the group comprising: calcium hydride, barium hydride, calcium carbide, borohydride such as sodium borohydride and potassium borohydride, sodium sulfide, ammonium salt, such as ammonium carbonate, ammonium sulfate, ammonium chloride, orthoester such as triethyl carbonate, tripropyl carbonate, NH₃BH₃, mixed anhydride of ion exchange resin with carboxylic groups and low-molecular carboxylic acid.

3. The method of claim 1 or 2, further comprising providing an acid, a base, or a catalyst promoting the reaction of non-volatile agent with blood.

4. The method of claim 3, wherein the base is included in the group of:
alkali carbonate, such as lithium carbonate, sodium carbonate, potassium carbonate;
alkali hydroxide, such as lithium hydroxide, sodium hydroxide, potassium hydroxide; di- and trialkali phosphate, such as disodium hydrogen phosphate and trisodium phosphate;
calcium hydroxide.

5. The method of claim 1, wherein the volatile agent is included in the group consisting of: hydrogen, acetylene, methanol, ethanol, hydrogen sulfide, ammonia, C1-C3 carboxylic acid.

6. A system for detecting bleeding from a wound, comprising:
a support (5) configured to be attached to a skin of a patient at or in the immediate vicinity of the wound (4);
a substantially non-volatile agent provided on the support (5), the non-volatile agent being disposed at or near the wound and being capable of forming a volatile agent on contact with blood;
**characterized in that** it further comprises:
a gas detection probe (8) for detection of the volatile agent wherein the probe is disposed at a distance from the support (5);
an electrically driven fan (13) for generating a flow of air in a direction from the support (5) to the probe (8); and
a guiding means for guiding said flow of air in a selected direction towards said probe, said guiding means comprising a tube (7), wherein the electrically driven fan (13) is mounted in the lumen of the tube (7);
whereby said probe (8) is configured to emit or increase or decrease an electric signal when contacted by the volatile agent as an indication of bleeding of the wound.

7. The system according to claim 6, wherein the non-volatile agent is included in the group consisting of: calcium hydride, barium hydride, calcium carbide, borohydride such as sodium borohydride and potassium borohydride, sodium sulfide, ammonium salt, such as ammonium carbonate, ammonium sulfate, ammonium chloride, orthoester such as triethyl carbonate, tripropyl carbonate, NH3BH3, mixed anhydride of ion exchange resin with carboxylic groups and low-molecular carboxylic acid.

8. The system according to claim 6 or 7, comprising any of acid, base, catalyst on a layer (43) of the support and said non-volatile agent on another layer (44) of the support.

9. The system according to claim 8, wherein the layer (53) comprising any of an acid, a base, or a catalyst is separated from the layer (55) comprising the non-volatile agent by an empty layer (54).

10. The according to claim 9, wherein the support is made of a textile material, wherein the textile material is included in the group of: cotton, linen, polymer fiber, cellulose fiber.

11. The system according to claim 9 or 10, comprising two or more layers of textile material.

12. The system according to claim 11, wherein two or more layers are bonded.

## Patentansprüche

1. Verfahren zum Nachweis von Blutungen aus einer Wunde, umfassend: Bereitstellen, auf einem Träger, eines im Wesentlichen nicht-flüchtigen Mittels, das in der Lage ist, bei Kontakt mit Blut ein flüchtiges Mittel zu bilden; Anordnen des nicht-flüchtigen Mittels auf dem Träger an oder nahe der Wunde; Bereitstellen einer Sonde zum Nachweis des flüchtigen Mittels; Anordnen der Sonde zum Nachweis des flüchtigen Mittels in einem Abstand von dem Träger; Erzeugen eines Luftstroms in einer Richtung von dem Träger zu der Sonde; Überwachen der Bildung eines flüchtigen Mittels durch die Sonde, wodurch eine Blutung der Wunde festgestellt wird.

2. Verfahren nach Anspruch 1, wobei das nicht-flüchtige Mittel in der Gruppe enthalten ist, umfassend: Calciumhydrid, Bariumhydrid, Calciumcarbid, Borhydrid wie Natriumborhydrid und Kaliumborhydrid, Natriumsulfid, Ammoniumsalz wie Ammoniumcarbonat, Ammoniumsulfat, Ammoniumchlorid, Orthoester wie Triethylcarbonat, Tripropylcarbonat, NH₃BH₃, gemischtes Anhydrid von lonenaustauscherharz mit Carboxylgruppen und niedermolekularer Carbonsäure.

3. Verfahren nach Anspruch 1 oder 2, weiterhin umfassend Bereitstellen einer Säure, einer Base oder eines Katalysators, der die Reaktion vom nichtflüchtigem Mittel mit Blut fördert.

4. Verfahren nach Anspruch 3, wobei die Base in der Gruppe enthalten ist von: Alkalicarbonat, wie Lithiumcarbonat, Natriumcarbonat, Kaliumcarbonat; Alkalihydroxid, wie Lithiumhydroxid, Natriumhydroxid, Kaliumhydroxid; Di- und Trialkaliphosphat, wie Dinatriumhydrogenphosphat und Trinatriumphosphat; Kalziumhydroxid.

5. Verfahren nach Anspruch 1, wobei das flüchtige Mittel in der Gruppe enthalten ist, bestehend aus Wasserstoff, Acetylen, Methanol, Ethanol, Schwefelwasserstoff, Ammoniak, C1-C3-Carbonsäure.

6. System zum Nachweis von Blutungen aus einer Wunde, umfassend:
einen Träger (5), ausgestaltet um an einer Haut eines Patienten an oder in der unmittelbaren Nähe der Wunde (4) befestigt zu werden;
ein im Wesentlichen nicht-flüchtiges Mittel, bereitgestellt auf dem Träger (5), wobei das nicht-flüchtige Mittel an oder Nahe der Wunde angeordnet ist und in der Lage ist, ein flüchtiges Mittel bei Kontakt mit Blut zu bilden;
**dadurch gekennzeichnet, dass** es weiterhin umfasst:
eine Gasnachweissonde (8) zum Nachweis des flüchtigen Mittels, wobei die Sonde in einem Abstand von dem Träger (5) angeordnet ist;
ein elektrisch angetriebenes Gebläse (13) zum Erzeugen eines Luftstroms in einer Richtung von dem Träger (5) zu der Sonde (8); und
ein Führungsmittel zum Führen des Luftstroms in einer gewählten Richtung zur Sonde, wobei das Führungsmittel ein Rohr (7) umfasst,
wobei das elektrisch angetriebene Gebläse (13) in dem Lumen des Rohrs (7) angeordnet ist;
wobei die Sonde (8) ausgestaltet ist, ein elektrisches Signal auszusenden oder zu erhöhen oder zu erniedrigen, wenn es durch das flüchtige Mittel als Hinweis auf Blutung der Wunde kontaktiert ist.

7. System nach Anspruch 6, wobei das nicht-flüchtige Mittel in der Gruppe enthalten ist, bestehend aus Calciumhydrid, Bariumhydrid, Calciumcarbid, Borhydrid wie Natriumborhydrid und Kaliumborhydrid, Natriumsulfid, Ammoniumsalz wie Ammoniumcarbonat, Ammoniumsulfat, Ammoniumchlorid, Orthoester wie Triethylcarbonat, Tripropylcarbonat, NH₃BH₃, gemischtes Anhydrid von lonenaustauscherharz mit Carboxylgruppen und niedermolekularer Carbonsäure.

8. System nach Anspruch 6 oder 7, umfassend eines aus Säure, Base, Katalysator auf einer Schicht (43) des Trägers und das nicht-flüchtige Mittel auf einer anderen Schicht (44) des Trägers.

9. System nach Anspruch 8, wobei die Schicht (53), die eines aus einer Säure, einer Base oder einem Katalysator enthält, von der Schicht (55), die das nicht-flüchtige Mittel enthält, durch eine leere Schicht (54) getrennt ist.

10. Das nach Anspruch 9, wobei der Träger aus einem Textilmaterial hergestellt ist, wobei das Textilmaterial enthalten ist, in der Gruppe aus: Baumwolle, Leinen, Polymerfasern, Zellulosefasern.

11. System nach Anspruch 9 oder 10, umfassend zwei oder mehr Schichten von Textilmaterial.

12. System nach Anspruch 11, wobei zwei oder mehr Schichten verbunden sind.

## Revendications

1. Procédé de détection de saignement d'une blessure, comprenant : la fourniture, sur un support, d'un agent sensiblement non volatil capable de former un agent volatil lors d'un contact avec du sang ; la mise en place de l'agent non volatil sur le support au niveau ou près de la blessure ; la fourniture d'une sonde pour la détection de l'agent volatil ; la mise en place de la sonde pour la détection de l'agent volatil à une distance du support ; la production d'un courant d'air dans une direction allant du support à la sonde ; la surveillance de la formation d'agent volatil par la sonde, ce qui détecte un saignement de la blessure.

2. Procédé selon la revendication 1, dans lequel l'agent non volatil est inclus dans le groupe comprenant : l'hydrure de calcium, l'hydrure de baryum, le carbure de calcium, un borohydrure tel que le borohydrure de sodium et le borohydrure de potassium, le sulfure de sodium, un sel d'ammonium tel que le carbonate d'ammonium, le sulfate d'ammonium, le chlorure d'ammonium, un orthoester tel que le carbonate de triéthyle, le carbonate de tripropyle, NH₃BH₃, un anhydride mixte de résine échangeuse d'ions avec des groupes carboxyliques et un acide carboxylique de faible masse moléculaire.

3. Procédé selon la revendication 1 ou 2, comprenant en outre la fourniture d'un acide, d'une base ou d'un catalyseur favorisant la réaction de l'agent non volatil avec du sang.

4. Procédé selon la revendication 3, dans lequel la base est incluse dans le groupe suivant : un carbonate de métal alcalin, tel que le carbonate de lithium, le carbonate de sodium, le carbonate de potassium ; un hydroxyde de métal alcalin, tel que l'hydroxyde de lithium, l'hydroxyde de sodium, l'hydroxyde de potassium ; un phosphate di- et tri-alcalin, tel que l'hydrogénophosphate disodique et le phosphate trisodique ; l'hydroxyde de calcium.

5. Procédé selon la revendication 1, dans lequel l'agent volatil est inclus dans le groupe constitué de : hydrogène, acétylène, méthanol, éthanol, sulfure d'hydrogène, ammoniac, acide carboxylique en C1 - C3.

6. Système pour détecter un saignement d'une blessure, comprenant :
un support (5) conçu pour être fixé à une peau d'un patient au niveau ou à proximité immédiate de la blessure (4) ;
un agent sensiblement non volatil fourni sur le support (5), l'agent non volatil étant disposé au niveau ou près de la blessure et étant capable de former un agent volatil lors d'un contact avec du sang ;
**caractérisé en ce qu'**il comprend en outre :
une sonde de détection de gaz (8) pour la détection de l'agent volatil, dans lequel la sonde est disposée à une distance du support (5) ;
un ventilateur à entraînement électrique (13) destiné à produire un courant d'air dans une direction allant du support (5) à la sonde (8) ; et
un moyen de guidage destiné à guider ledit courant d'air dans une direction choisie allant vers ladite sonde, ledit moyen de guidage comprenant un tube (7), dans lequel le ventilateur à entraînement électrique (13) est monté dans la lumière du tube (7) ;
moyennant quoi ladite sonde (8) est conçue pour émettre ou augmenter ou diminuer un signal électrique lorsqu'elle est touchée par l'agent volatil en tant qu'indication de saignement de la blessure .

7. Système selon la revendication 6, dans lequel l'agent non volatil est inclus dans le groupe constitué de : l'hydrure de calcium, l'hydrure de baryum, le carbure de calcium, un borohydrure tel que le borohydrure de sodium et le borohydrure de potassium, le sulfure de sodium, un sel d'ammonium tel que le carbonate d'ammonium, le sulfate d'ammonium, le chlorure d'ammonium, un orthoester tel que le carbonate de triéthyle, le carbonate de tripropyle, NH₃BH₃, un anhydride mixte de résine échangeuse d'ions avec des groupes carboxyliques et un acide carboxylique de faible masse moléculaire.

8. Système selon la revendication 6 ou 7, comprenant l'un quelconque d'un acide, d'une base, d'un catalyseur sur une couche (43) du support et ledit agent non volatil sur une autre couche (44) du support.

9. Système selon la revendication 8, dans lequel la couche (53) comprenant l'un quelconque d'un acide, d'une base ou d'un catalyseur est séparée de la couche (55) comprenant l'agent non volatil par une couche vide (54).

10. Le selon la revendication 9, dans lequel le support est constitué d'un matériau textile, dans lequel le matériau textile est inclus dans le groupe suivant : coton, lin, fibre polymère, fibre de cellulose.

11. Système selon la revendication 9 ou 10, comprenant deux couches ou plus de matériau textile.

12. Système selon la revendication 11, dans lequel deux couches ou plus sont liées.
